(19) Europäisches Patentamt
European Patent Office
Office européen des brevets

(11) Veröffentlichungsnummer: **0 256 468**
**A2**

(12) # EUROPÄISCHE PATENTANMELDUNG

(21) Anmeldenummer: **87111531.7**

(22) Anmeldetag: **10.08.87**

(51) Int. Cl.[4]: **C07D 239/46**, C07D 401/12, A61K 7/00

(30) Priorität: **18.08.86 DE 3627922**

(43) Veröffentlichungstag der Anmeldung:
**24.02.88 Patentblatt 88/08**

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Rose, David, Dr.**
**Holbeinweg 7**
**D-4010 Hilden(DE)**
Erfinder: **Lieske, Edgar**
**Hunsrückenstrasse 40**
**D-4000 Düsseldorf(DE)**
Erfinder: **Maak, Norbert, Dr.**
**Im Jagdfeld 41 a**
**D-4040 Neuss(DE)**

(54) **Neue Tetraaminopyrimidin-Derivate und deren Verwendung in Haarfärbemitteln.**

(57) Neue 2,4,5,6-Tetraaminopyrimidin-Derivate der allgemeinen Formel

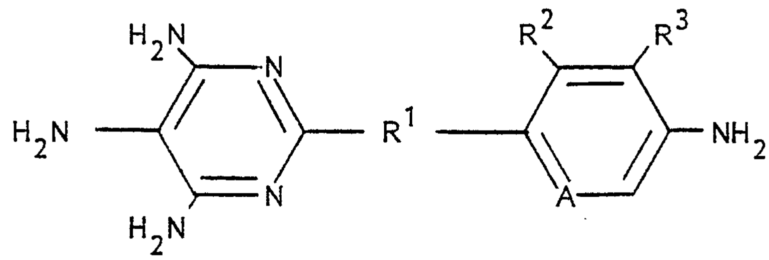

in der $R^1$ eine Gruppe $-NH-(CH_2)_n-NH-$, worin n = 2 - 4 ist, eine Gruppe $-NH-CH_2-CH(OH)-CH_2-NH-$oder eine Gruppe

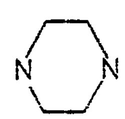

ist, $R^2$ und $R^3$ unabhängig voneinander Wasserstoff, Chlor oder eine Gruppe $-OR^4$ sind und A eine CH-Gruppe oder ein Stickstoffatom ist und deren Salze eignen sich als Entwicklerkomponenten für Oxidationshaarfärbemittel, die mit einer Vielzahl üblicher Kupplerverbindungen ein breites Spektrum brillanter Farbnuancen mit gutem Aufziehvermögen auf menschlichem Haar und hohen Echtheitseigenschaften ausbilden.

EP 0 256 468 A2

## "Neue Tetraaminopyrimidin-Derivate und deren Verwendung in Haarfärbemitteln"

Gegenstand der Erfindung sind neue Derivate des 2,4,5,6-Tetraaminopyrimidins und deren Salze sowie deren Verwendung als Entwicklerkomponente in Oxidationshaarfärbemitteln.

Für das Färben von Haaren spielen die sogenannten Oxidationshaarfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Eine bestimmte Entwicklersubstanz kann durch Kombination mit unterschiedlichen Kupplern auch sehr unterschiedliche Farbnuancen bilden. Trotzdem gelingt es oft nicht, mit Hilfe einer einzigen Entwicklersubstanz zu der Vielzahl natürlicher Haarfarbnuancen zu kommen. In der Praxis ist daher meist eine Kombination verschiedener Entwicklerkomponenten und Kupplerkomponenten erforderlich, um eine einzige, natürlich wirkende Haarfärbung zu erhalten.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Auf ziehvermögen auf menschlichem Haar besitzen, wobei keine merklichen Unterschiede zwischen strapaziertem und frisch nachgewachsenem Haar bestehen dürfen. Sie sollen beständig sein gegen Licht, Wärme und den Einfluß chemischer Reduktionsmittel, z. B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Als Entwicklersubstanzen werden üblicherweise primäre aromatische Amine mit einer weiteren in Para- oder Orthoposition befindlichen freien oder substituierten Hydroxy-oder Aminogruppe, ferner Diaminopyridinderivate, heterocyclische Hydrazonderivate und 4-Aminopyrazolonderivate eingesetzt. Als sogenannte Kupplersubstanzen werden m-Phenylendiaminderivate, Naphthole, Resorcinderivate und Pyrazolone verwendet.

Aus der deutschen Offenlegungsschrift 23 59 393 war die Verwendung von 2,4,5,6-Tetraaminopyrimidinen als Entwicklerkomponente in Haarfärbemitteln bekannt. Die dort beschriebenen 2,4,5,6-Tetraaminopyrimidin-derivate liefern mit einer Vielzahl von Kupplern nur ein begrenztes Spektrum von Nuancen vorwiegend im Bereich gelber, brauner und roter Farbtöne.

Gegenstand der Erfindung sind neue 2,4,5,6-Tetraaminopyrimidin-Derivate der Formel (I)

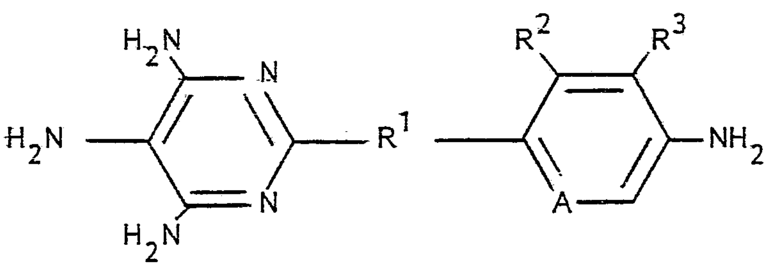

( I )

in der $R^1$ eine Gruppe $-NH-(CH_2)_n-NH-$, worin n = 2 - 4 ist, eine Gruppe $-NH-CH_2-CH(OH)-CH_2-NH-$oder eine Gruppe

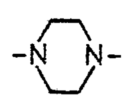

ist, $R^2$ und $R^3$ unabhängig voneinander Wasserstoff, Chlor oder eine Gruppe $-OR^4$ ist, worin $R^4$ eine Alkylgruppe mit 1 bis 4 C-Atomen ist und A eine CH-Gruppe oder ein Stickstoffatom ist und deren Salze.

Diese neuen 2,4,5,6-Tetraaminopyrimidin-Derivate der Formel I sind Oxidationsfarbstoffvorprodukte vom Typ der Entwicklersubstanzen, d. h. sie vermögen unter der Einwirkung von Oxidationsmitteln Farbstoffe auszubilden. In Gegenwart von Kupplersubstanzen werden jedoch besonders brillante und intensive Farben gebildet. Als Kupplersubstanzen eignen sich vor allem die m-Phenylendiamine, m-Aminophenole, Resorcine, Naphthol 1, 1,5-und 2,7-Dihydroxynaphthalin, Hydroxy-und Aminopyridine, Hydroxychinoline und Aminopyrazolone. Mit diesen und anderen bekannten Kupplersubstanzen bilden die erfindungsgemäßen 2,4,5,6-Tetraaminopyrimidin-Derivate ein breites Spektrum von Nuancen das von orangegelb bis tiefblau reicht. Vor allem aber werden mit vielen Kupplern Farbnuancen erzeugt, die sonst nur unter Verwendung von zwei oder mehr verschiedenen Entwicklern zugänglich sind. Die erfindungsgemäßen 2,4,5,6-Tetraaminopyrimidin-Derivate der Formel I eignen sich daher hervorragend zur Verwendung als Oxidationsfarbstoffvorprodukte vom Entwickler-Typ in Haarfärbemitteln. Haarfärbemittel, welche die erfindungsgemäßen 2,4,5,6-Tetraaminopyrimidin-Derivate als Entwicklerkomponente neben üblichen Kupplerkomponenten enthalten, zeigen ein besonders gleichmäßiges Aufziehvermögen auf strapaziertem wie auf frisch nachgewachsenem Haar. Die erhaltenen Haaranfärbungen weisen eine hohe Thermostabilität auf. Bevorzugt, insbesondere wegen ihrer leichten Zugänglichkeit, sind die 2,4,5,6-Tetraaminopyrimidin-Derivate der Formel I, in welchen $R^2$ und $R^3$ Wasserstoff sind, wenn A ein Stickstoffatom ist und in welchen wenigstens eine der Gruppen $R^2$ und $R^3$ Wasserstoff ist, wenn A eine CH-Gruppe ist. Aufgrund des hohen Molekulargewichtes sind die neuen 2,4,5,6,-Tetraaminopyrimidin-Derivate wenig resorbierbar, was sich günstig auf die toxikologischen und dermatologischen Eigenschaften auswirkt.

Die erfindungsgemäßen 2,4,5,6-Tetraaminopyrimidin-Derivate der Formel I lassen sich dadurch herstellen, daß man Verbindungen der Formel II

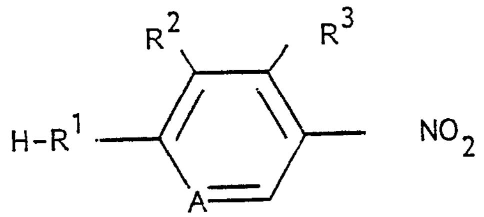

(II)

mit 2-Methylmercapto-4,6-diamino-5-nitroso-pyrimidin zur Umsetzung bringt und die erhaltenen Verbindungen der Formel (III) (Zwischenstufe)

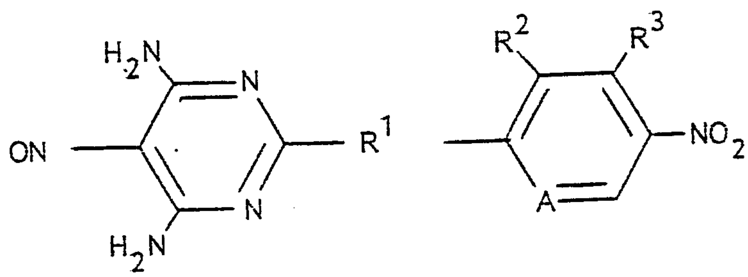

(III)

katalytisch hydriert. In den Formel II und III haben $R^1$, $R^2$, $R^3$ und A die gleiche Bedeutung wie in Formel I.

Die erfindungsgemäßen 2,4,5,6-Tetraaminopyrimidin-Derivate können entweder als solche oder in Form ihrer Salze mit anorganischen oder organischen Säuren, z. B. als Hydrochloride, Sulfate, Phosphate, Acetate, Propionate, Lactate oder Citrate isoliert und in Haarfärbemittel eingesetzt werden.

Ein weiterer Erfindungsgegenstand sind Haarfärbemittel mit einem Gehalt an Oxidationsfarbstoffvorprodukten in einem kosmetischen Träger, die als Oxidationsfarbstoffvorprodukte 2,4,5,6-Tetraaminopyrimidin-Derivate der Formel I als Entwicklerkomponente in einer Menge von 0,05 bis 10 Millimol pro 100 g neben üblichen Kupplerkomponenten und gegebenenfalls direktziehenden Haarfarbstoffen enthalten.

In den erfindungsgemäßen Haarfärbemitteln werden die Entwicklersubstanzen und die Kupplersubstanzen im allgemeinen in äquimolaren Mengen eingesetzt, ein gewisser Überschuß einzelner Oxidationsfarbstoffvorprodukte ist jedoch nicht nachteilig, so daß Entwicklersubstanzen und Kupplersubstanzen in einem Molverhältnis von 1 : 0,5 bis 1 : 2 eingesetzt werden können. Bei einem leichten Überschuß der Kupplerverbindung bilden die erfindungsgemäßen Haarfärbemittel etwas intensivere Färbenuancen aus. Es ist nicht erforderlich, daß die 2,4,5,6-Tetraaminopyrimidin-Derivate der Formel I einheitliche Verbindungen sind. Vielmehr können auch Gemische dieser Verbindungen zum Einsatz gelangen.

Zur Modifikation der Haaranfärbung können den erfindungsgemäßen Haarfärbmitteln auch bekannte direktziehende Haarfarbstoffe, z. B. Nitrophenylendiaminderivate, Anthrachinonfarbstoffe oder Indophenole zugesetzt werden.

Zur Herstellung der erfindungsgemäßen Haarfärbemittel werden die Oxidationsfarbstoffvorprodukte und gegebenenfalls direktziehende Farbstoffe in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz-und Emulgiermittel wie anionische, nichtionische oder ampholytische Tenside, z. B. Fettalkholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungs mittel wie z. B. Methyl-oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Die Anwendung der erfindungsgemäßen Haarfärbemittel kann, unabhängig von der Art der kosmetischen Zubereitung, z. B. als Creme, Gel oder Shampoo im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen.

Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

Die nachfolgenden Beispiele sollen den Erfindungsgegenstand näher erläutern, ohne ihn jedoch hierauf zu beschränken.

## Beispiele

### 1. Herstellungsbeispiele

Allgemeine Vorschrift:

#### Stufe 1

Eine Mischung aus 0,05 Mol der Verbindung der Formel II und 0,05 Mol 2-Methylmercapto-4,6-diamino-5-nitrosopyrimidin wurde in 500 ml Ethanol 5 Stunden unter Rückfluß zum Sieden erhitzt. Nach dem Abkühlen auf 20 °C wurde das Zwischenprodukt der Formel III abfiltriert und im Trockenschrank bei 80 °C getrocknet.

#### Stufe 2

5 g des Zwischenproduktes der Formel III wurden in 200 ml Ethanol gelöst und nach Zugabe von 0,5 g Palladium auf Aktivkohle (10%ig) bei 20 °C und 2 bar Wasserstoffdruck katalytisch hydriert. Nach Beendigung der Wasserstoffaufnahme wurde der Katalysator abfiltriert, das Filtrat mit konzentrierter Salzsäure angesäuert und zur Trockene eingedampft. Dabei wurde das 2,4,5,6-Tetraaminopyrimidin-Derivat der Formel I erhalten.

Nach dieser allgemeinen Herstellvorschrift wurden die folgenden Verbindungen hergestellt (Tabelle I).

Tabelle I

| Verbindung der Formel I | $R^1$ | $R^2$ | $R^3$ | A |
|---|---|---|---|---|
| 1.1 | $-NH-(CH_2)_n-NH-$ | H | H | CH |
| 1.2 | $-NH-(CH_2)_3-NH-$ | H | H | CH |
| 1.3 | $-NH-(CH_2)_4-NH-$ | H | H | CH |
| 1.4 | $-NH-CH_2-CH-(OH)-CH_2-NH-$ | H | H | CH |
| 1.5 | $-NH-(CH_2)_2-NH$ | Cl | H | CH |
| 1.6 | $-NH-(CH_2)_2-NH$ | H | $OCH_3$ | CH |
| 1.7 | 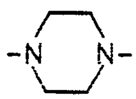 | H | H | CH |
| 1.8 | $-NH-(CH_2)_2-NH$ | H | H | N |

Die chemischen Bezeichnungen der Verbindungen 1.1 bis 1.8, deren Aussehen und Schmelzpunkte, die Ausgangsverbindungen (II), aus der sie hergestellt wurden und die Zwischenstufen (III), die dabei erhalten wurden:

1.1 2-[ 2-(p-Aminoanilino)-ethylamino ] -4,5,6-Diaminopyrimidin-tetrahydrochlorid
Hellgelbe Kristalle, Schmelzpunkt 187 °C (unter Zersetzung)

Ausgangsverbindung (II)
N-(p-Nitrophenyl)-ethylendiamin
Zwischenstufe (III)
2-[ 2-(p-Nitroanilino)-ethylamino ] -4,6-diamino-5-nitrosopyrimidin
Orange Kristalle, Schmelzpunkt 279 - 282 °C

1.2 2-[ 3-(p-Aminoanilino)-propylamino ] -4,5,6-triaminopyrimidin-tetrahydrochlorid
Farblose Kristalle, Schmelzpunkt 180 °C (unter Zersetzung)

Ausgangsverbindung (II)
N-(p-Nitrophenyl)-trimethylendiamin
Zwischenstufe (III)
2-[ 3-(p-Nitroanilino)-propylamino ] -4,6-diamino-5-nitrosopyrimidin
Orangebraune Kristalle, Schmelzpunkt 263 °C

1.3 2-[ 4-(p-Aminoanilino)-butylamino ] -4,5,6-triaminopyrimidin-tetrahydrochlorid
Farblose Kristalle, Schmelzpunkt ab 170 °C (unter Zersetzung)

Ausgangsverbindung (II)
N-(p-Nitrophenyl)-tetramethylendiamin

Zwischenstufe (III)
2-[ 4-(p-Nitroanilino)-butylamino ] -4,6-diamino-5-nitrosopyrimidin
Ockerfarbene Kristalle, Schmelzpunkt 210 - 216 °C

1.4 2-[ 3-(p-Aminoanilino)-2-hydroxypropylamino ] -4,5,6-triaminopyrimidin-tetrahydrochlorid
Beige Kristalle, Schmelzpunkt ab 175 °C (unter Zersetzung)

Ausgangsverbindung (II)
N-(p-Nitrophenyl)-2-hydroxy-1,3-diaminopropan
Zwischenstufe (III)
2-[ 3-(p-Nitroanilino)-2-hydroxypropylamino ] -4,6-diamino-5-nitrosopyrimidin
Orangebraune Kristalle, Schmelzpunkt 236 - 246 °C

1.5 2-[ 2-(2-Chlor-4-aminoanilino)-ehtylamino ] -4,5,6-triaminopyrimidin-tetrahydrochlorid

Ausgangsverbindung (II)
N-(O-Chlor-p-nitrophenyl)-ethylendiamin
Zwischenstufe (III)
2-[ 2-(2-Chlor-4-nitroanilino)-ethylamino ] -4,6-diamino-5-nitrosopyrimidin
Orangegelbe Kristalle, Schmelzpunkt ab 258 °C (unter Zersetzung)

1.6 2-[ 2-(3-Methoxy-4-aminoanilino)-ethylamino ] -4,5,6-triaminopyrimidin-tetrahydrochlorid

Ausgangsverbindung (II)
N-(4-Nitro-3-methoxyphenyl)-ethylendiamin
Zwischenstufe (III)
2-[ 2-(3-Methoxy-4-nitroanilino)-ethylamino ] -4,6-diamino-5-nitrosopyrimidin
Ockerfarbene Kristalle, Schmelzpunkt ab 205 °C (unter Zersetzung)

1.7 2-[ 4-p-Aminophenylpiperazinyl-(1) ] -4,5,6-triamino-pyrimidin-tetrahydrochlorid
gelbe Kristalle, Schmelzpunkt ab 190 °C

Ausgangsverbindung (I)
1-(4-Nitrophenyl)-piperazin
Zwischenstufe (III)
2-[ 4-p-Nitrophenylpiperazinyl-(1) ] -4,6-diamino-5-nitrosopyrimidin
Rote Kristalle, Schmelzpunkt 283 - 289 °C

1.8 2-[ 2-(5-Aminopyridyl(2)-amino)-ethylamino ] -4,5,6-triaminopyrimidin-tetrahydrochlorid
Beige Kristalle, Schmelzpunkt ab 178 °C (unter Zersetzung)

Ausgangsverbindung (II)
2-(ß-Aminoethylamino)-5-nitropyridin
Zwischenstufe (III)
2-[ 2-(5-Nitropyridyl(2)-amino)-ethylamino ] -4,6,-diamino-5-nitroso-pyrimidin
Orange Kristalle, Schmelzpunkt über 310 °C

## 2. Anwendungsbeispiele

Es wurden erfindungsgemäße Haarfärbemittel in Form einer Haarfärbe-Cremeemulsion der folgenden Zusammensetzung hergestellt:

Fettalkohol $C_{12-14}$ 10,0 g
Fettalkohol $C_{12-14}$ + 2 EO-sulfat, Na-Salz, 28%ig 25,0 g
Wasser 60,0 g
Tetraaminopyrimidin-Derivat 7,5 mMol
Kupplerkomponente 7,5 mMol
$Na_2SO_3$ (Inhibitor) 1,0 g
konzentrierte Ammoniak-Lösung bis pH = 9,5
Wasser ad 100 g

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Entwickler wurden die Tetraaminopyrimidin-Derivate gemäß Beispiel 1.1 bis 1.8 eingesetzt. Als Kupplerkomponenten wurden die folgenden Verbindungen verwendet:

2.1 1-Naphthol
2.2 2,4-Diaminophenoxy-ethanol
2.3 2,4-Diaminophenetol
2.4 2-Chlor-6-methyl-3-aminophenol
2.5 1,3-Bis-(2,4-diaminophenoxy)-propan
2.6 2,4-Dichlor-3-aminophenol
2.7 5-Amino-2-methylphenol
2.8 5-Amino-4-Chlor-2-methylphenol
2.9 m-Aminophenol
2.10 1-Phenyl-3-aminopyrazolon-5
2.11 3-Amino-2-methylamino-6-methoxypyridin
2.12 6-Hydroxychinolin
2.13 2,6-Dihydroxy-3,4-dimethylpyridin
2.14 2,7-Dihydroxynaphtahlin
2.15 2,6-Dihydroxypyridin
2.16 2-Amino-3-hydroxypyridin
2.17 4,6-Dichlorresorcin
2.18 2-Methylresorcin

Die mit den genannten Oxidationsfärbemittelvorprodukten erhaltenen Haaranfärbungen sind der Tabelle II zu entnehmen.

Tabelle II

| Anwendungs-beispiel | Entwickler-komponente | Kuppler-komponente | erhaltener Farbton |
|---|---|---|---|
| A1 | 1.1 | 2.1 | mattblau |
| A2 | 1.1 | 2.2 | dunkelblau |
| A3 | 1.1 | 2.3 | dunkelblau |
| A4 | 1.1 | 2.6 | graublau |
| A5 | 1.1 | 2.8 | dunkelviolett |
| A6 | 1.1 | 2.9 | dunkelviolett |
| A7 | 1.1 | 2.10 | mahagonirot |
| A8 | 1.1 | 2.11 | dunkelgrün |
| A9 | 1.1 | 2.12 | oliv |
| A10 | 1.1 | 2.13 | oliv |
| A11 | 1.1 | 2.14 | olivgelb |
| A12 | 1.1 | 2.15 | olivbraun |
| A13 | 1.1 | 2.16 | graubraun |
| A14 | 1.1 | 2.17 | rotbraun |

| Anwendungs-beispiel | Entwickler-komponente | Kuppler-komponente | erhaltener Farbton |
|---|---|---|---|
| A15 | 1.2 | 2.4 | dunkelblau |
| A16 | 1.2 | 2.14 | bronzebraun |
| A17 | 1.2 | 2.18 | burgunderrot |
| A18 | 1.3 | 2.4 | dunkelblau |
| A19 | 1.3 | 2.14 | haarbraun |
| A20 | 1.3 | 2.18 | burgunderrot |
| A21 | 1.4 | 2.5 | mattblau |
| A22 | 1.4 | 2.7 | graurubin |
| A23 | 1.4 | 2.13 | oliv |
| A24 | 1.4 | 2.14 | oliv |
| A25 | 1.5 | 2.7 | graublau |
| A26 | 1.5 | 2.14 | gelbbraun |
| A27 | 1.5 | 2.18 | graurubin |
| A28 | 1.6 | 2.8 | graublau |
| A29 | 1.6 | 2.10 | grauviolett |
| A30 | 1.7 | 2.6 | graublau |
| A31 | 1.7 | 2.9 | grauviolett |
| A32 | 1.8 | 2.1 | graublau |
| A33 | 1.8 | 2.18 | graubraun |

**Ansprüche**

1. 2,4,5,6-Tetraaminopyrimidin-Derivate der allgemeinen Formel (I)

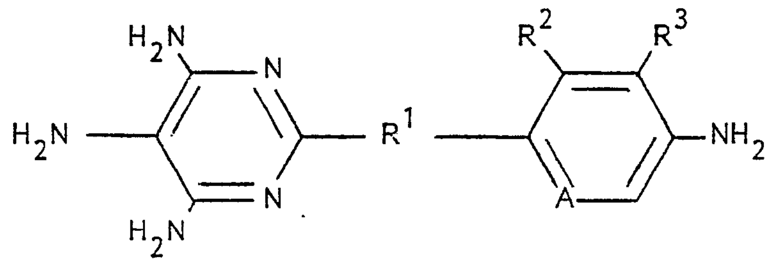

(I)

in der $R^1$ eine Gruppe $-NH-(CH_2)_n-NH-$, worin n = 2 - 4 ist, eine Gruppe $-NH-CH_2-CH(OH)-CH_2-NH-$oder eine Gruppe

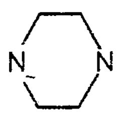

ist, $R^2$ und $R^3$ unabhängig voneinander Wasserstoff, Chlor oder eine Gruppe $-OR^4$ ist, worin $R^4$ eine Alkylgruppe mit 1 bis 4 C-Atomen ist und A eine CH-Gruppe oder ein Stickstoffatom ist und deren Salze.

2. 2,4,5,6-Tetraaminopyrimidin-Derivate gemäß Anspruch 1, dadurch gekennzeichnet, daß $R^2$ und $R^3$ Wasserstoff sind, wenn A ein Stickstoffatom ist und daß wenigstens eine der Gruppen $R^2$ und $R^3$ Wasserstoff ist, wenn A eine CH-Gruppe ist.

3. Verfahren zur Herstellung von 2,4,5,6-Tetraaminopyrimidin-Derivaten der allgemeinen Formel I

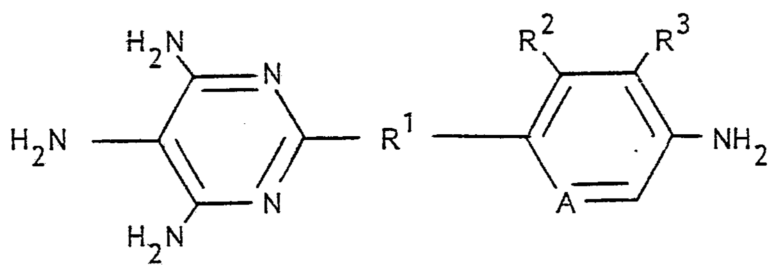

(I)

in der $R^1$ eine Gruppe $-NH-(CH_2)_n-NH-$, worin n = 2 - 4 ist, eine Gruppe $-NH-CH_2-CH(OH)-CH_2-NH-$oder eine Gruppe

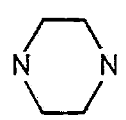

ist, $R^2$ und $R^3$ unabhängig voneinander Wasserstoff, Chlor oder eine Gruppe $-OR^4$ ist, worin $R^4$ eine Alkylgruppe mit 1 bis 4 C-Atomen ist und A eine CH-Gruppe oder ein Stickstoffatom ist, dadurch gekennzeichnet, daß man Verbindungen der Formel II

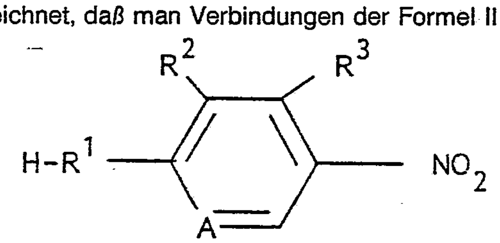

(II)

mit 2-Methylmercapto-4,6-diamino-5-nitroso-pyrimidin zur Umsetzung bringt und die erhatlenen Verbindungen der Formel (III)

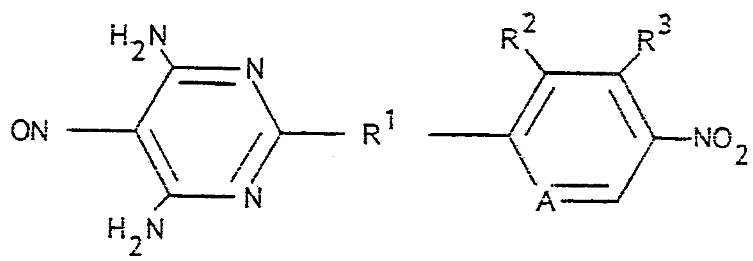

(III)

katalytisch hydriert.

4. Verwendung der 2,4,5,6-Tetraaminopyrimidin-Derivate der Formel I nach den Ansprüchen 1 oder 2 als Oxidationsfarbstoffvorprodukte vom Entwicklertyp in Haarfärbemitteln.

5. Haarfärbemittel, enthaltend Oxidationsfarbstoffvorprodukte in einem kosmetischen Träger, dadurch gekennzeichnet, daß als Oxidationsfarbstoffvorprodukte 2,4,5,6-Tetraaminopyrimidin-Derivate der Formel I nach den Ansprüchen 1 oder 2 als Entwicklerkomponente in einer Menge von 0,05 bis 10 Millimol pro 100 g neben üblichen Kupplerkomponenten und gegebenenfalls direktziehenden Haarfarbstoffen enthalten sind.